# NEUE EUROPÄISCHE PATENTSCHRIFT

(11) **EP 1 121 600 B2**
(45) Veröffentlichungstag und Bekanntmachung des Hinweises auf die Entscheidung über den Einspruch: **25.06.2014**
(45) Hinweis auf die Patenterteilung: 15.03.2006
(21) Anmeldenummer: 99953805.1
(22) Anmeldetag: 13.10.1999
(51) Int. Cl.: G01N 33/74, C07K 14/585, A61K 38/23, A61K 38/22

(54) **VERFAHREN UND SUBSTANZEN FÜR DIE DIAGNOSE UND THERAPIE VON SEPSIS UND SEPSISÄHNLICHEN SYSTEMISCHEN INFEKTIONEN**
METHOD AND SUBSTANCES FOR DIAGNOSIS AND THERAPY OF SEPSIS AND SEPSIS-LIKE SYSTEMIC INFECTIONS
PROCEDE ET SUBSTANCES POUR LE DIAGNOSTIC ET LE TRAITEMENT D'UNE SEPTICEMIE ET D'INFECTIONS GENERALISEES SEMBLABLES A UNE SEPTICEMIE

(30) Priorität: 15.10.1998 DE 19847690
(43) Veröffentlichungstag der Anmeldung: 08.08.2001
(60) Teilanmeldung: 03028136.4
(73) Patentinhaber: B.R.A.H.M.S Aktiengesellschaft, 16761 Hennigsdorf (DE)
(72) Erfinder: BERGMANN, Andreas, D-12351 Berlin (DE); STRUCK, Joachim, D-12161 Berlin (DE); WEGLÖHNER, Wolfgang, D-12099 Berlin (DE)
(74) Vertreter: Kilger, Ute
(86) Internationale Anmeldenummer: PCT/EP1999/007692
(87) Internationale Veröffentlichungsnummer: WO 2000/022439

(56) Entgegenhaltungen:
- DE-C- 4 227 454
- OKAHARA S ET AL: "SERUM PRO-GASTRIN-RELEASING PEPTIDE (PROGRP) IN SUBJECTS WITH OR WITHOUT HERICOBACTER PYLORI INFECTION" GASTROENTEROLOGY,US,ELSEVIER, NEW YORK, NY,, Bd. 110, Nr. 4, April 1996 (1996-04), Seite A217 XP000884904 ISSN: 0016-5085
- NORIHARU SHIJUBO ET AL: "ELEVATED PROGASTRIN-RELEASING PEPTIDE (31-98) CONCENTRATIONS IN PLEURAL EFFUSIONS DUE TO SMALL-CELL LUNG CARCINOMA" RESPIRATION,CH,KARGER, BASEL, Bd. 63, Nr. 2, 1. März 1996 (1996-03-01), Seiten 106-110, XP000608937 ISSN: 0025-7931
- MIYAKE, Y. ET AL.: "Pro-Gastrin-Releasing Peptide(31-98) Is a Specific Tumor Marker in Patients with Small Cell Lung Carcinoma" CANCER RESEARCH, Bd. 54, 15. April 1994 (1994-04-15), Seiten 2136-2140, XP002132024
- GUO Y ET AL: "PRODUCTION OF ENDOTHELINS BY THE VENTILATORY MUSCLES IN SPETIC SHOCK" AMERICAN JOURNAL OF RESPIRATORY CELL AND MOLECULAR BIOLOGY,US,AMERICAN LUNG ASSOCIATION, NEW YORK, NY, Bd. 1, Nr. 3, September 1998 (1998-09), Seiten 470-476, XP000879419 ISSN: 1044-1549
- MCLOUGHLIN L ET AL: "CHARACTERIZATION OF CIRCULATING PRO-OPIOMELANCOCORTIN-RELATED PEPTIDES IN HUMAN SEPTIC SHOCK" JOURNAL OF ENDOCRINOLOGY,GB,BRISTOL, Bd. 119, Nr. 1, Oktober 1988 (1988-10), Seiten 159-165, XP000879345 ISSN: 0022-0795 in der Anmeldung erwähnt
- NYLEN E S ET AL: "PNEUMONITIS-ASSOCIATED HYPERPROCALCITONINEMIA" AMERICAN JOURNAL OF MEDICAL SCIENCES,XX,XX, Bd. 312, Nr. 1, Juli 1996 (1996-07), Seiten 12-18, XP000879269 ISSN: 0002-9629
- ASSICOT M ET AL: "HIGH SERUM PROCALCITONIN CONCENTRATIONS IN PATIENTS WITH SEPSIS AND INFECTION" LANCET THE,GB,LANCET LIMITED. LONDON, Bd. 341, Nr. 8844, 27. Februar 1993 (1993-02-27), Seiten 515-518, XP000371780 ISSN: 0140-6736
- KARZAI W ET AL: "PROCALCITONIN - A NEW INDICATOR OF THE SYSTEMIC RESPONSE TO SEVERE INFECTIONS" INFECTION,DE,MMV MEDIZIN VERLAG, MUENCHEN, Bd. 25, Nr. 6, November 1997 (1997-11), Seiten 329-334, XP000879253 ISSN: 0300-8126
- QURESHI, N.U. ET AL.: "Endogenous neuropeptide Y mediates vasoconstriction during endotoxic and hemorrhagic shock" REGULATORY PEPTIDES, Bd. 75-76, 25. September 1998 (1998-09-25), Seiten 215-220, XP000925467
- NORLANDER, T. ET AL.: "Effects of experimental Mycoplasma pulmonis infection on sensory neuropeptides and airway mucosa in the rat" EUROPEAN RESPIRATORY JOURNAL, Bd. 10, 1997, Seiten 2334-2342, XP000925422
- KÜLLERTZ, G.: "Die Bedeutung der Aktivitätsbestimmung des Enzyms Dipeptidyl-Peptidase IV (DP IV) im klinischen Laboratorium" LAB.MED., Bd. 12, 1988, Seiten 123-130, XP000933866
- VANHAM, G. ET AL.: "Decreased Expression of the Memory Marker CD26 on Both CD4+ and CD8+ T Lymphocytes of HIV-Infected Subjects" JOURNAL OF ACQUIRED IMMUNE DEFICIENCY SYNDROMES, Bd. 6, Nr. 7, Juli 1993 (1993-07), Seiten 749-757, XP000925435
- YARON, A. AND NAIDER, F.: "Proline-Dependent Structural and Biological Properties of Peptides and Proteins" CRITICAL REVIEWS IN BIOCHEMISTRY AND MOLECULAR BIOLOGY, Bd. 28, 1993, Seiten 31-81, XP000925565
- ORAVECZ T ET AL: "REGULATION OF THE RECEPTOR SPECIFICITY AND FUNCTION OF THE CHEMOKINE RANTES (REGULATED ON ACTIVATION, NORMAL T CELL EXPRESSED AND SECRETED) BY DIPEPTIDYL PEPTIDASE IV (CD26)-MEDIATED CLEAVAGE" JOURNAL OF EXPERIMENTAL MEDICINE,JP,TOKYO, Bd. 186, Nr. 11, 1. Dezember 1997 (1997-12-01), Seiten 1865-1872, XP002056059 ISSN: 0022-1007 in der Anmeldung erwähnt
- P PROOST ET AL: "Amino-terminal truncation of chemokines by CD26/Dipeptidyl-peptidase IV" JOURNAL OF BIOLOGICAL CHEMISTRY,US,AMERICAN SOCIETY OF BIOLOGICAL CHEMISTS, BALTIMORE, MD, Bd. 273, Nr. 13, 27. März 1998 (1998-03-27), Seiten 7222-7227-7227, XP002102982 ISSN: 0021-9258 in der Anmeldung erwähnt
- WRENGER, S. ET AL: "Amino-terminal truncation of procalcitonin, a marker for systemic bacterial infections, by dipeptidyl peptidase IV (DP IV)." FEBS LETTERS, (JAN. 21, 2000) VOL. 466, NO. 1, PP. 155-159., XP002143500

## Beschreibung

Die vorliegende Erfindung betrifft neue Diagnose möglichkeiten, die sich aus neuen, experimentell abgesicherten Erkenntnissen im Zusammenhang mit dem Auftreten von Procalcitonin bzw. Procalcitonin-Teilpeptiden bei Sepsis und sepsisähnlichen schweren systemischen Infektionen ableiten ließen.

Aus den Patenten DE 42 27 454 bzw. EP 0 656 121 B1 bzw. US 5,-639, 617 ist es bekannt, daß die Bestimmung des Prohormons Procalcitonin bzw. von sich davon ableitenden Teilpeptiden in einem Serum oder Plasma eines Patienten, bei dem ein Sepsisrisiko besteht bzw. bei dem sepsistypische Krankheitserscheinungen festgestellt werden, ein wertvolles diagnostisches Hilfsmittel zur Früherkennung, d.h. zur Erkennung von Infektionen, die zu Sepsis führen können, und deren Abgrenzung von nicht-infektiösen Ätiologien, zur Erkennung des Schweregrads und zur therapiebegleitenden Verlaufsbeurteilung von Sepsis und sepsisähnlichen systemischen Infektionen darstellt. Besonders wertvoll hat sich die genannte Bestimmung zur differentialdiagnostischen Unterscheidung von Krankheitserscheinungen, die auf systemische mikrobielle Infektionen zurückzuführen sind, von anderen Krankheitserscheinungen nicht-infektiöser Ätiologie erwiesen, die aufgrund ihres klinischen Erscheinungsbildes auf eine Sepsis hindeuten könnten, jedoch in Wirklichkeit nicht auf eine systemische mikrobielle Infektion zurückzuführen sind, z.B. von Krankheitserscheinungen, die auf nicht-infektiöse Entzündungen einzelner Organe, auf postoperative Abstoßungsreaktionen oder Krebserkrankungen zurückzuführen sind. Auch können systemische Entzündungen von lokalen abgegrenzt werden.

Bezüglich eines Überblicks über den jüngeren Erkenntnisstand wird verwiesen auf W. Karzai et al. in Infection, Vol. 25 (1997), 6, S. 329-334 und die darin zitierte bzw. erwähnte weitere Fachliteratur.

Procalcitonin ist bekanntgeworden als Prohormon von Calcitonin, und seine vollständige Aminosäuresequenz ist seit längerem bekannt (FEBS 167 (1984), S. 93-97). Procalcitonin wird unter normalen Umständen in den C-Zellen der Schilddrüse produziert und dann spezifisch in das Hormon Calcitonin sowie die weiteren Teilpeptide Katacalcin und einen N-terminalen Rest aus 57 Aminosäuren ("Aminoprocalcitonin") gespalten.

Da bei Sepsis stark erhöhte Procalcitonin-Spiegel auch bei Patienten beobachtet werden, denen die Schilddrüse völlig entfernt wurde, mußte der Schluß gezogen werden, daß das im Blut von Sepsispatienten nachweisbare Procalcitonin außerhalb der Schilddrüse gebildet wird, wobei bezüglich der Organe bzw. Zellen oder der Gewebe, die für die Procalcitonin-Produktion bei Sepsis bestimmend sind, in der Fachliteratur unterschiedliche Vermutungen, die teilweise durch experimentelles Material gestützt wurden, geäußert wurden.

Was die Natur des bei Sepsis als "Procalcitonin" bestimmten Peptids angeht, so wurde zwar in den o.g. Patenten von Anfang an klargestellt, daß das bestimmte Peptid nicht völlig mit dem bekannten Procalcitonin-Peptid voller Länge identisch sein muß, das in den Schilddrüsen als Calcitonin-Vorläufer gebildet wird. Die Frage, ob sich das im Falle von Sepsis gebildete Procalcitonin von dem in den Schilddrüsen gebildeten Procalcitonin unterscheidet, blieb jedoch bisher ungeklärt. Als mögliche Unterschiede waren posttranslationale Modifikationen des bekannten Procalcitonins wie Glykosylierungen, Phosphorylierungen oder Veränderungen der Primärstruktur denkbar, jedoch auch modifizierte, verkürzte oder verlängerte Aminosäuresequenzen. Da die bisher angewandten analytischen Bestimmungsverfahren keine Unterschiede zwischen dem als calcitonin-Vorläufer bekannten Procalcitonin und dem bei Sepsis gebildeten Procalcitonin erkennen ließen, wurde vorläufig allgemein davon ausgegangen, daß das bei Sepsis gebildete Procalcitonin mit dem Calcitonin-vorläufer identisch ist und somit ein Peptid mit der bekannten Procalcitonin-Sequenz von 116 Aminosäuren (Procalcitonin 1-116) darstellt.

Wie die nachfolgend im experimentellen Teil dieser Anmeldung genauer erläuterten Bestimmungen im Labor der Anmelderin ergeben haben, unterscheidet sich das bei Sepsis gebildete Procalcitonin jedoch zwar geringfügig, jedoch signifikant von dem in der Schilddrüse gebildeten vollständigen Procalcitonin 1-116. Aus den festgestellten Unterschieden ergaben sich dann eine Reihe von wissenschaftlichen Schlußfolgerungen, die in neue diagnostische und therapeutische Verfahren, darin verwendbare Substanzen bzw. weiterverfolgbare wissenschaftliche Ansätze umgesetzt werden konnten.

Ausgangspunkt für den in der vorliegenden Patentanmeldung offenbarten Erfindungskomplex ist die überraschende Erkenntnis, daß das bei Sepsis und sepsisähnlichen systemischen Infektionen im Serum von Patienten in vergleichsweise hohen Konzentrationen nachweisbare Procalcitonin nicht das vollständige Procalcitonin 1-116 mit 116 Aminosäuren ist, sondern ein am Aminoterminus um ein Dipeptid verkürztes, ansonsten jedoch identisches Procalcitonin mit einer Aminosäuresequenz von nur 114 Aminosäuren (Procalcitonin 3-116).

Das gegenüber dem vollständigen Procalcitonin fehlende Dipeptid weist die Struktur Ala-Pro auf. Das Fehlen eines Dipeptids mit einem Prolinrest als zweite Aminosäure des Amino-Terminus des vollständigen Procalcitoninsequenz gab Anlaß zu der Vermutung, daß bei der Erzeugung des bei Sepsis nachweisbaren Procalcitonins 3-116 möglicherweise eine bestimmte Peptidase eine Rolle spielt, und zwar die sogenannte Dipeptidyl-(Amino)-Peptidase IV (DP IV oder DAP IV bzw. CD26).

Zur Bestimmung einer möglichen Rolle der Dipeptidyl-Aminopeptidase IV im Rahmen von systemischen Infektionen bzw. von Sepsis haben die Erfinder daher experimentell geprüft, ob eine Korrelation der physiologischen DAP IV Konzentrationen mit dem Nachweis einer Sepsis möglich ist. Die erhaltenen Ergebnissen zeigten eine derartige Korrelation.

Aus den erhaltenen genaueren Ergebnissen wurde ferner eine Hypothese entwickelt, daß.das Auftreten hoher Procalcitonin-Konzentrationen bei Sepsis und systemischen Infektionen möglicherweise keine isolierte Erscheinung ist, sondern daß in ähnlicher Weise auch erhöhte Konzentrationen anderer Prohormone meßbar sein könnten, so daß die Bestimmung derartiger Prohormone eine mögliche Alternative zur Procalcitonin-Bestimmung darstellt bzw. geeignet ist, die Procalcitonin-Bestimmung in Einzelfällen zu ergänzen oder in diagnostisch signifikanter Weise weiter abzusichern.

Es ist Aufgabe der vorliegenden Erfindung, neue Verfahren für die in vitro Sepsisdiagnostik anzugeben, die die für diesen Zweck etablierte Bestimmung des Prohormons Procalcitonin in Serum oder Plasma ersetzen oder ergänzen können.

Diese Aufgabe wird gemäß Anspruch 1 durch Verfahren zur differentialdiagnostischen Früherkennung und Erkennung, zur Beurteilung des Schweregrads und zur therapiebegleitenden Verlaufsbeurteilung von Sepsis gelöst, bei denen man in einer Probe eines Serums aus dem Blut eines Patienten den Gehalt mindestens eines Peptid-Prohormons, das nicht Procalcitonin ist, bestimmt und aus der festgestellten Menge des bestimmten Prohormons auf das Vorliegen einer Sepsis, ihren Schweregrad und/oder den Erfolg einer therapeutischen Behandlung schließt.

Vorteilhafte Ausgestaltungen sind in den Unteransprüchen 2 bis 7 wiedergegeben.

Nachfolgend wird unter Bezugnahme auf mehrere Diagramme ausgewähltes experimentelles Material vorgelegt, das die neuen Erkenntnisse untermauert bzw. das für die Richtigkeit der daraus abgeleiteten Annahmen spricht.

In den Figuren zeigen:
- Figur 1: die Ergebnisse einer Procalcitonin-Isolierung und Aufreinigung per HPLC aus einem Mischserum aus gesammelten Seren von verschiedenen Patienten mit schwerer Sepsis;
- Figur 2: die Ergebnisse einer massenspektroskopischen Untersuchung derjenigen Fraktionen des Mischserums aus Fig.1, die eine hohe Procalcitonin-Immunreaktivität aufwiesen;
- Figur 3: Ergebnisse der Bestimmung der Enzymaktivität von Dipeptidyl-Aminopeptidase IV in septischen Seren und Normalseren; sowie
- Figur 4: die Ergebnisse der Bestimmung von Procalcitonin in septischen Seren und Normalseren in Gegenüberstellung mit Ergebnissen der Bestimmung eines weiteren Prohormons, nämlich pro-Gastrin-Releasing-Peptide (proGRP), in den gleichen Seren.
- Figur 5: die Ergebnisse der Bestimmung von Procalcitonin in Seren eines Kollektivs von 20 Normalpersonen sowie andererseits 20 Sepsispatienten.
- Figur 6: die Ergebnisse der Bestimmung von Pro-ANF (in pg/Röhrchen) in den gleichen Kollektiven von Normalpersonen und Sepsispatienten wie in Figur 5.
- Figur 7: die Ergebnisse der Bestimmung von Pro-ADM (in pg/Röhrchen) in den gleichen Kollektiven von Normalpersonen und Sepsispatienten wie in Figur 5.
- Figur 8: die Ergebnisse der Bestimmung von Pro-END (in pg/Röhrchen) in den gleichen Kollektiven von Normalpersonen und Sepsispatienten wie in Figur 5.
- Figur 9.: die Ergebnisse der Bestimmung von Pro-BNP (in pg/Röhrchen) in den gleichen Kollektiven von Normalpersonen und Sepsispätienten wie in Figur 5.

### EXPERIMENTELLER TEIL

### A. Isolierung und Charakterisierung des endogenen Procalcitonin-Peptids aus Seren septischer Patienten

Durch Vermischen von Serumproben von verschiedenen Patienten mit schwerer Sepsis wurde ein Mischserum mit einem Gesamtvolumen von 68 ml hergestellt. Die Procalcitonin-Konzentration in dem erhaltenen Mischserum wurde mit Hilfe eines kommerziellen Procalcitonin-Assays (LUMItest PCT, B.R.A.H.M.S Diagnostica) zu 280 ng/ml (Gesamtmenge 19µg) ermittelt. Das Mischserum wurde mit einem gleichen Volumen eines Puffers (68 ml; 10mM EDTA, 1 mg/ml Maus-IgG, 2 mg/ml Schaf-IgG, 2 mg/ml bovines IgG, 0,1 mMol Leupeptin, 50 µM Amastatin in PBS) vermischt, und das in der Probe enthaltene Procalcitonin wurde affinitätschromatographisch isoliert und gereinigt.

Hierzu wurde die gesamte Mischprobe bei einer Durchflußrate von 0,5 ml/min viermal hintereinander über eine Affinitätssäule (0,5 x 1 cm, Anti-Calcitonin-Antikörper, gebunden an Carbolink der Firma Pierce, Procalcitonin-Bindekapazität ca. 20 µg) gepumpt. Anschließend wurde die Säule mit 30 ml PBS gewaschen, und das gebundene Peptid wurde mit Hilfe von 50 mM Essigsäure (pH ca. 2,0) eluiert. Der Säulenausfluß wurde kontinuierlich auf Absorption bei 280 nm überwacht, und die von der Essigsäure eluierte Proteinfraktion wurde aufgefangen (Endvolumen 2,0 ml).

Das auf diese Weise gesammelte Material wurde über Umkehrphasen-HPLC an einer rpC₁₈-Säule *µ* Bondapak 0,4 x 30 mm (Firma Waters) gereinigt. Die Durchflußgeschwindigkeit betrug 1 ml/min, Laufmittel und Elutionsbedingungen sind der folgenden Tabelle 1 zu entnehmen.

**Tabelle 1: Elutionsbedingungen rp-HPLC Procalcitonin**

| | | | |
|---|---|---|---|
| Laufmittel A: | 5 % Acetonitril | | |
| | 20 mM NH₄-Acetat | | |
| Laufmittel B: | 90 % Acetonitril | | |
| | 20 mM NH₄-Acetat | | |
| Gradient: | 0,0 min | 100 % A | 0 % B |
| | 2,5 min | 100 % A | 0 % B |
| | 5,0 min | 89 % A | 11 % B |
| | 55,0 min | 56 % A | 44 % B |
| | 60,0 min | 0 % A | 100 % B |

Der Säulenausfluß wurde kontinuierlich auf seine Absorption bei 214 nm vermessen und in Fraktionen von 0,25 ml gesammelt. Mit Hilfe eines handelsübliche Procalcitonin-Assays (LUMItest PCT, B.R.A.H.M.S Diagnostica) wurden diejenigen Fraktionen bestimmt, in denen eine PCT-Immunreaktivität nachweisbar war. Es zeigte sich, daß die Haupt-Immunreaktivität in der 51. Fraktion als scharfe Bande eluiert wurde. Außerdem wurden heterogen zusammengesetzte Proteinfraktionen mit geringeren PCT-Immunreaktivitäten in den Fraktionen 39 bis 49 erhalten.

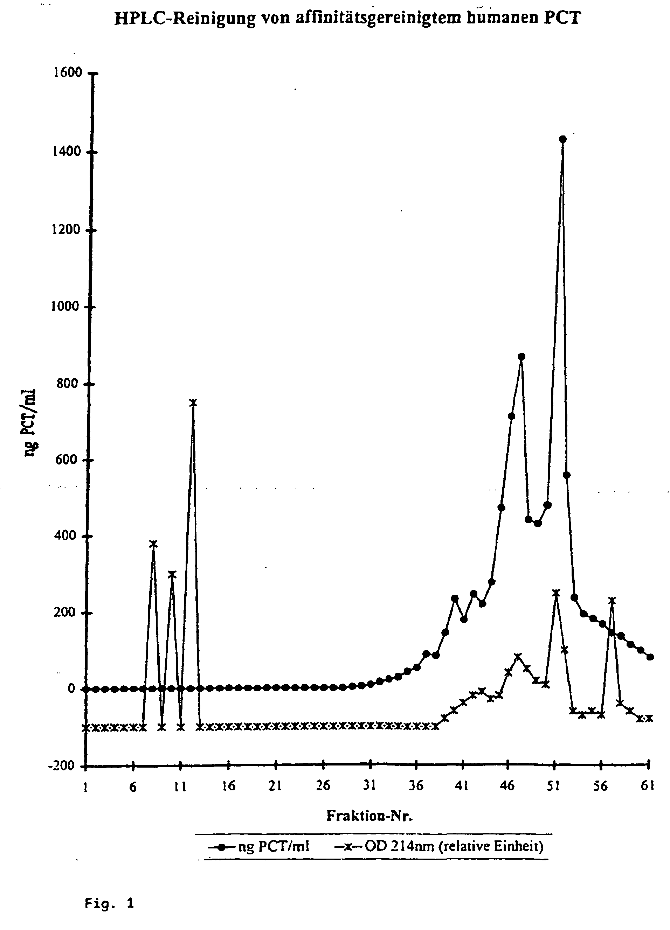

Figur 1 zeigt die ermittelte PCT-Immunreaktivität (ausgedrückt als ng PCT/ml) für die einzelnen gesammelten Fraktionen der rp HPLC-Chromatographie, überlagert einer Kurve, die die optische Dichte (OD) der eluierten Fraktionen darstellt.

Alle Fraktionen, die eine positive Procalcitonin-Immunreaktivität aufwiesen, wurden durch Begasen mit Stickstoff getrocknet. Anschließend wurden die Proben massenspektrometrisch analysiert und einer N-terminalen Sequenzierung unterworfen.

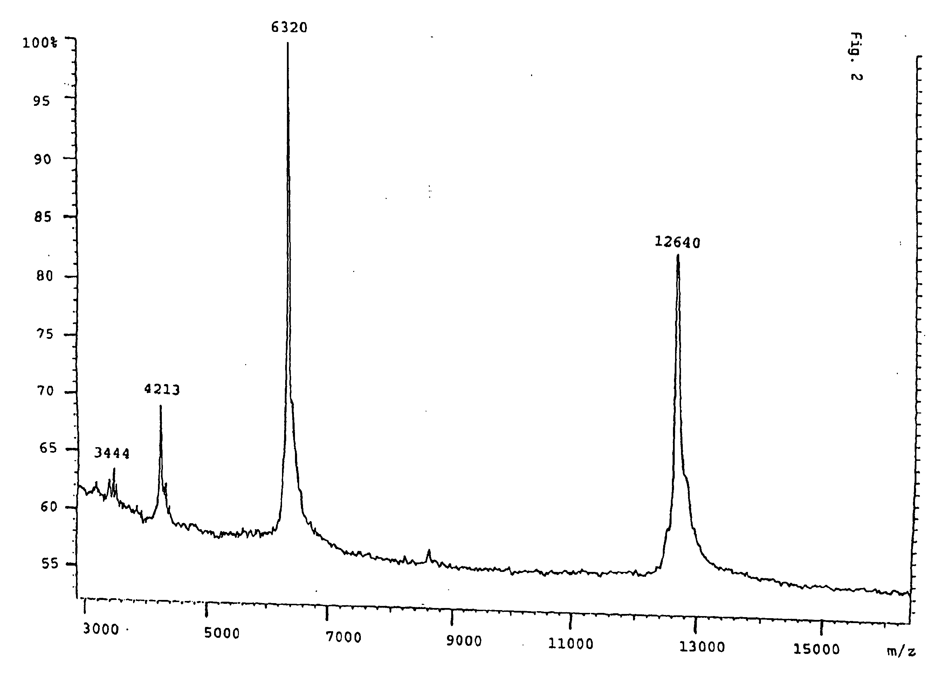

Bei der massenspektrometrischen Untersuchung (MALDI-TOF-Methode) wurde für die Fraktionen 50-52 das in Figur 2 gezeigte Profil erhalten, aus dem sich eine Molmasse von 12640 ±15 ergab. Sämtliche anderen massenspektrometrisch untersuchten Fraktionen (36-49, 53-59) ergaben heterogene Massenverteilungen mit Molmassen <12640. Ihre Einzelmasse ergab im Vergleich zur Intensität der Masse der Fraktion 50-52 eine Intensität von <2 %. Damit wurde nachgewiesen, daß die Procalcitonin-Immunreaktivität in Seren von Sepsispatienten mit einer Masse von 12640 ±15 assoziiert ist. Keine der gewonnenen Fraktionen wies eine höhere Masse auf.

Die in den Fraktionen 36-59 enthaltenen Peptide wurden einer N-terminalen Sequenzierung unterworfen. Auch hierbei erwies sich der Inhalt der Fraktionen 36-49 und 53-59 als heterogen, d.h. es wurde eine Vielzahl von unterschiedlichen N-Termini ermittelt.

Für die Fraktion 50-52, in denen die überwiegende Procalcitonin-Immunreaktivität zu finden war, ergab sich, daß die darin enthaltenen Peptide eindeutig folgenden N-Terminus (15 Aminosäuren) aufwiesen:
Phe Arg Ser Ala Leu Glu Ser Ser Pro Ala Asp Pro Ala Thr Leu

Das Peptid aus den Fraktionen 50-52 wurde anschließend mittels Protease Glu-C bzw. Trypsin verdaut, die entstandenen Fragmente wurden auf an sich bekannte Weise über SMART-HPLC gewonnen und anschließend massenspektrometrisch und sequenzanalytisch untersucht.

Es wurde eine Sequenz erhalten, die vollständig mit der Sequenz der Aminosäuren 3-116 des bekannten Procalcitonins 1-116 übereinstimmte. Die theoretische Masse dieser Sequenz betrug 12627, was mit der massenspektrometrisch ermittelten Masse von 12640±15 übereinstimmt.

Damit wurde nachgewiesen, daß im Blut von Sepsispatienten ein Procalcitonin-Peptid zirkuliert, das 114 Aminosäuren umfaßt und als Procalcitonin 3-116 zu bezeichnen ist. Das Peptid ist nicht durch posttranslationale Modifikationen wie Phosphorylierungen oder Glykosylierungen verändert.

Das Procalcitonin 3-116 wurde bisher in der wissenschaftlichen Literatur noch nicht als mögliches endogenes Procalcitonin-Teilpeptid diskutiert, und es bestand daher bisher für den Fachmann auch noch keinerlei Veranlassung, dieses Peptid gezielt herzustellen und auf seine Eigenschaften zu untersuchen. Die obigen Befunde lieferten nunmehr jedoch einen Anlaß zur gezielten Herstellung des genannten Procalcitonin 3-116 nach gentechnologischen Techniken. Seine Herstellung wird nachfolgend beschrieben.

### B. Klonierung, Expression und Aufreinigung von rekombinantem Procalcitonin 3-116.

### 1. Klonierung

Die für Procalcitonin 3-116 (nachfolgend abgekürzt PCT 114) codierenden DNA-Fragmente wurden unter Anwendung einer PCR-Amplifikation mit Hilfe geeigneter Oligonukleotidprimer aus einem humanen Schilddrüsen-cDNA-Pool isoliert. Das gewünschte Fragmente wurde mittels gängiger Methoden (Ausubel FM, Brent R, Kingston RE, Moore DD, Seidman JG, Smith JA, und Struhl K (1991), Current Protocols in Molecular Biology, John Wiley & Sons, New York) kloniert, und die korrekte Nukleotidsequenz wurde durch DNA-Sequenzierung und Vergleich mit der bekannten, für Procalcitonin codierenden DNA-Sequenz verifiziert.

Für die Expression der für PCT 114 codierenden cDNA wurde ein Vektor verwendet, der neben einem T7-Promotor eine Region enthält, die für das Signalpeptid des sog. pelB-Proteins codiert. Dieses pelB-Signalpeptid sorgt dafür, daß ein nach Klonierung und Expression entstandenes Fusionsprotein durch die cytoplasmatische Membran der für die Expression verwendeten Wirtzellen in den periplasmatischen Raum transportiert wird. Bei diesem Transportvorgang wird gleichzeitig das N-terminale Signalpeptid durch eine membranständige Signalpeptidase abgetrennt (Stader JA und Silhavy TJ (1990), Engineering Escherichia coli to secrete heterologous gene products, Methods Enzymol. 185, 166-187). Diese Vorgehensweise garantiert, daß das gefundene Expressionsprodukt exakt die gewünschte Sequenz aufweist. Bei dieser Vorgehensweise fehlt das bei anderen Methoden zur Expression notwendige N-terminale Methionin.

Nach der Klonierung der cDNA für PCT 114 in einen Vektor der genannten Art und der Transformierung von E. coli mit diesem Vektor wurde Procalcitonin 3-116 exprimiert. Die periplasmatische Fraktion mit dem exprimierten Procalcitonin 3-116 wurde auf an sich bekannte Weise isoliert (Neu HC und Heppel LA (1965), The release of enzymes from Escherichia coli by osmotic shock and during the formation of spheroblasts, J. Biol. Chem. 240, 3685-3692). Nach einer Zentrifugierung (100.000 g, 30 min, 4°C) und Filtration des flüssigen Überstands (0,2 µm) wurde das erhaltene Filtrat durch Anionen-Austauschchromatographie aufgetrennt. Die Fraktionen mit Procalcitonin-Immunreaktivität wurden vereinigt und über Umkehrphasen-HPLC aufgereinigt, wie im Zusammenhang mit der Isolierung von PCT 3-116 aus septischen Seren beschrieben wurde.

Alle Fraktionen mit Procalcitonin-Immunreaktivität wurden vereinigt und lyophilisiert. Wie eine Überprüfung des Materials mittels SDS-PAGE ergab, war das so gewonnene Material zu mindestens 95% sauber.

Die Identität des exprimierten und aufgereinigten Peptids als Procalcitonin 3-116 wurde durch Massenspektrometrie und Sequenzanalyse bestätigt.

Das erhaltene rekombinante Procalcitonin 3-116 stellt ein neues rekombinantes Peptid dar und kann in dieser Form zur Herstellung von Immunreagenzien verwendet sowie auf seine Eignung als Therapeutikum bzw. auf seiner Fähigkeit, im Sinne der o.g. Veröffentlichung (Eric S Nylen, a.a.O.) prophylaktisch und therapeutisch zu wirken, untersucht werden.

Zur Herstellung von Kalibratoren für PCT-Assays wurde das oben für die gentechnologische Herstellung von Procalcitonin 3-116 beschriebene Verfahren in im wesentlichen identischer Form auch zur Herstellung des vollständigen Procalcitonins 1-116 und des Procalcitonins 2-116 eingesetzt.

### C. Ermittlung der Dipeptidyl-Aminopeptidase IV (DAP IV)-Aktivität in humanen Normalseren bzw. Seren von Patienten mit schwerer Sepsis

Jeweils 20 Serumproben von gesunden Normalpersonen sowie von Sepsispatienten wurden auf ihre für Dipeptidyl-Aminopeptidase IV-spezifische Enzymaktivität untersucht. Die DAP IV-Enzymaktivität wurde auf an sich bekannte Weise fluorometrisch unter Verwendung von Lys-Pro-4-Methoxy-beta-naphthylamid vermessen. Dazu wurden jeweils 2 µl des zu überprüfenden Serums mit 3 ml Substrat (50 µg/ml Lys-Pro-4-Methoxy-beta-naphylamid, 50 mM Tris/HCl, pH 7,5) vermischt, und die auftretende Fluoreszenz wurde kontinuierlich unter Anregung mit Licht einer Wellenlänge von 340 nm bei einer Emissionswellenlänge von 410 nm gemessen. Das Fluoreszenzsignal wurde mittels einer 4-Methoxy-naphthylamin-Lösung kalibriert.
Die auf diese Weise ermittelte Enzymaktivität wird in nmol/min angegeben.

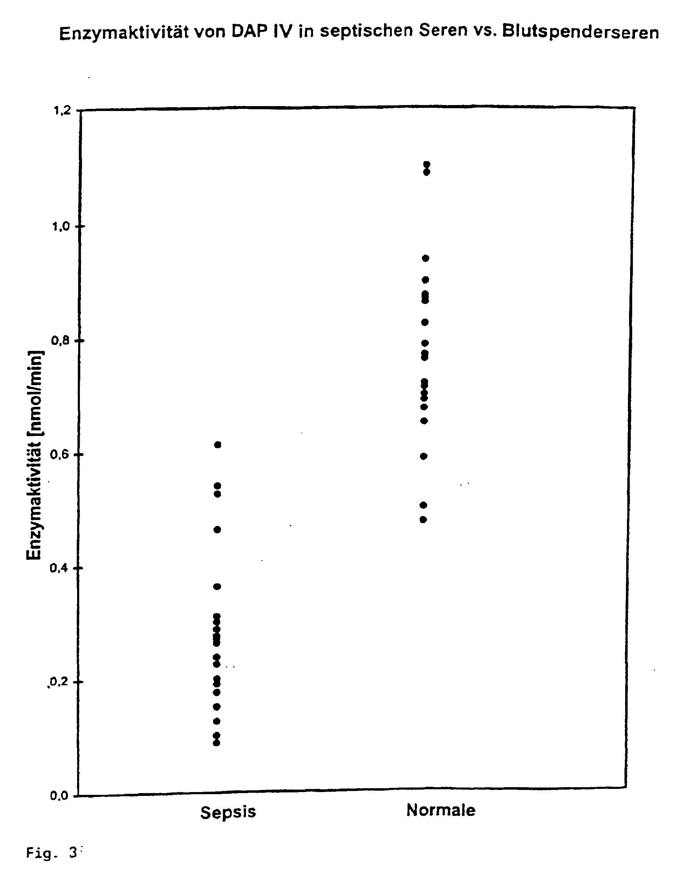

Die erhaltenen Ergebnisse sind Figur 3 dargestellt.

Es ist klar zu erkennen, daß die DAP IV-Enzymaktivität in den Sepsis-Seren deutlich niedriger liegt als in den Seren von gesunden Normalpersonen (Blutspenderseren). Damit läßt sich die Bestimmung der DAP IV-Enzymaktivität im Plasma oder Serum auch zur Erkennung von Sepsis in Patientenseren heranziehen.

Die bei Sepsis deutlich erniedrigte Plasmakonzentration an DAP IV kann einerseits als Beleg dafür angesehen werden, daß DAP IV an einem Sepsis-Geschehen mitbeteiligt ist. Andererseits deuten die Ergebnisse darauf hin, daß es nicht die Konzentration an DAP IV im Plasma sein kann, die für die Bildung von Procalcitonin 3-116 verantwortlich ist. Die erhaltenen Ergebnisse legen vielmehr den Schluß nahe, daß Procalcitonin 3-116 durch gewebs- bzw. zellgebundene DAP IV, möglicherweise intrazellulär, gebildet und aus Procalcitonin-erzeugenden oder speichernden Zellen freigesetzt wird.

Die der Literatur entnehmbare Information, daß DAP IV von aktivierten T-Zellen exprimiert wird, (vergleiche Hegen und Oravecz, Protein-Reviews on the WEB; Fleischer, a.a.O.) deutet auf einen engen Zusammenhang zwischen der Expression von DAP IV und dem Aktivierungszustand des Immunsystems hin, das bei einer septischen systemischen Infektion extrem belastet ist und daher typische Reaktionen zeigt, die sich u.a. in einer stark erhöhten Procalcitonin 3-116 Bildung äußern.

Abgesehen von den sich aus den obigen Befunden ergebenden Möglichkeiten, DAP IV im Rahmen der Sepsis-Diagnose zu bestimmen, können die obigen Ergebnisse auch darauf hindeuten, daß die bei einer Sepsis kaskadenartig ablaufenden Vorgänge durch DAP IV-Hemmstoffe therapeutisch zu beeinflußt werden können, wodurch es möglich sein könnte, die Freisetzung von Procalcitonin 3-116 sowie anderen Hormonen oder konvertierten Prohormonen unter Sepsis zu verhindern oder zu vermindern, so daß pathologische Konsequenzen dieser Prohormonfreisetzung vermindert oder vermieden werden können.

### D. Bestimmung der Konzentrationen anderer Prohormone als Procalcitonin bei Sepsis

Die Tatsache, daß bei Sepsis nicht das vollständige Prohormon Procalcitonin, sondern ein modifiziertes, um ein Xaa-Pro-Dipeptid verkürzte Prohormon freigesetzt wird, führte zu der Hypothese, daß bei Sepsis nicht nur Procalcitonin 3-116 freigesetzt wird, sondern daß Procalcitonin 3-116 vielleicht nur ein Vertreter aus einer ganzen Gruppe von Prohormonen oder ähnlichen Peptiden, z.B. solchen mit immunmodulatorischen Eigenschaften, ist, die bei Sepsis in erhöhtem Maße und möglicherweise konvertierter Form freigesetzt werden

Eine Überprüfung bekannter Prohormone und der für diese Prohormone der Literatur entnehmbaren Aminosäuresequenzen zeigte, daß tatsächlich bei den meisten bekannten Prohormonen am Aminoterminus ein Dipeptid zu finden ist, das als Xaa-Pro definiert werden kann und das daher in einem ähnlichen Sinne, wie beim Procalcitonin beobachtet, abgespalten werden kann. Im einzelnen finden sich am Aminoterminus sehr vieler Prohormone oder Immunmodulatoren Dipeptide des genannten Typs. Die nachfolgende tabellarische Übersicht über Literaturdaten gibt einen Überblick über einige ausgewählte Prohormone bzw. Immunmodulatoren, das bei diesen zu am Amino-Terminus zu findende Dipeptid sowie ihre Gesamtzahl der Aminosäuren.

Die in der Tabelle 2 aufgeführten Prohormone stellen Beispiele für Prohormone dar, deren Konzentrationen bei Sepsis erhöht sein können, ohne daß die Aufzählung als erschöpfend anzusehen ist. Bei den Immunmodulatoren ist mit einer Beeinflussung der Aktivität durch Abspaltung eines Dipeptids zu rechnen.

**Tabelle 2: Pro-Hormon/ Dipeptid am Gesamtzahl Immunmodulator N-Terminus aller Aminosäuren**

| | | |
|---|---|---|
| pro-Endothelin-1 (pro-END) | Ala Pro | 195 |
| pro-Brain-Natriuretic-Peptide (pro-BNP) | His Pro | 108 |
| pro-Atrial-Natriuretic-Peptide (pro-ANP; auch pro-Atrionatriuretischer Faktor, pro-ANF) | Asn Pro | 128 |
| pro-Leptin | Val Pro | 146 |
| pro-Neuropeptide Y | Tyr Pro | 69 |
| pro-Somatostatin | Ala Pro | 92 |
| pro-Neuropeptide YY | Thr Pro | 69 |
| Interleukin 6 | Val Pro | 183 |
| Interleukin 10 | Ser Pro | 160 |
| pro-Gastrin-Releasing Peptid (proGRP) | Val Pro | 115 |
| pro-Opiomelanocortin | Trp Cys | 241 |
| pro-Adrenomedullin (pro-ADM) | Ala Arg | 164 |
| Procalcitonin (PCT) | Ala Pro | 116 |

Die bisherigen experimentellen Befunde deuten tatsächlich darauf hin, daß bei einer systemischen Infektion wie Sepsis generell Prohormone und/oder Peptid-Immunmodulatoren wie Interleukine, möglicherweise unter Modifizierung durch Abspaltung von Dipeptiden am Amino-Terminus, freigesetzt werden und daß diese durch Wechselwirkung mit zugehörigen spezifischen Rezeptoren oder anderen Bindern möglicherweise weitere Folgeschritte in der Kaskade einer Immunantwort auslösen.

Es wurde parallel zu einer Procalcitonin-Bestimmung in Normalseren und Seren von Sepsis-Patienten auch die Bestimmung weiterer Prohormone durchgeführt, die eher willkürlich ausgewählt ausgewählt worden waren. Es waren dies (i) das pro-Gastrin-Releasing Peptide (proGRP), (ii) das pro-Atrial-Natriuretic-Peptide (pro-ANP bzw. pro-ANF), (iii) das pro-Adrenomedullin (pro-ADM), (iv) das pro-Endothelin (pro-FND) und (v) das pro-Brain-Natriuretic-Peptide (pro-BNP).

### D.1. Bestimmung von proGRP in Seren von Sepsis-Patienten und von Normalpersonen

Für die Bestimmung von proGRP ist ein Assay im Handel. ProGRP wird in einer jüngeren Veröffentlichung als Tumormarker beim kleinzelligen Bronchialkarzinom beschrieben (Petra Stieber et al, J Lab Med 1997, 21(6):336-344). Der Assay zur Bestimmung von ProGRP wird von der Tonen Corporation unter der Bezeichnung ProGRP ELISA KIT™ im Handel angeboten.

Im Abstract von Okahara et al. aus Gastroenterology, Bd. 110, Nr.4, April 1996, Seite A217 wird berichtet von einer relativ geringen proGRP-Erhöhung bei einer H.pylori-(Helicobacter pylori) Infektion auf das ca. 1,5 fache gegenüber fastenden Nichtinfizierten. H.pylori-Infektionen sind in der Bevölkerung sehr weit verbreitete lokale Infektionen des Magens ohne Beziehung zu Sepsis, die keine übliche Komplikation einer H.pylori-Infektion ist.

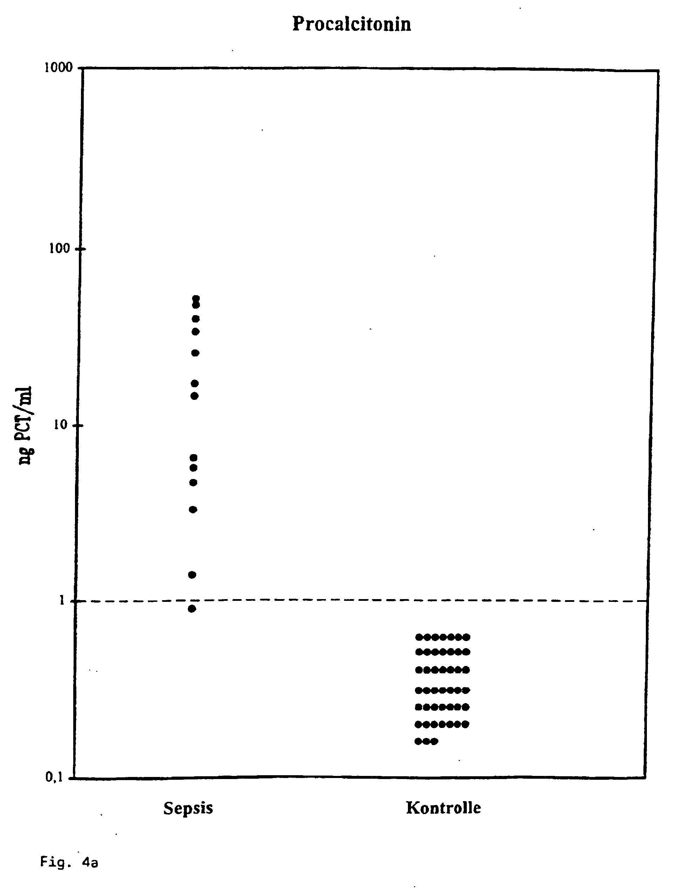

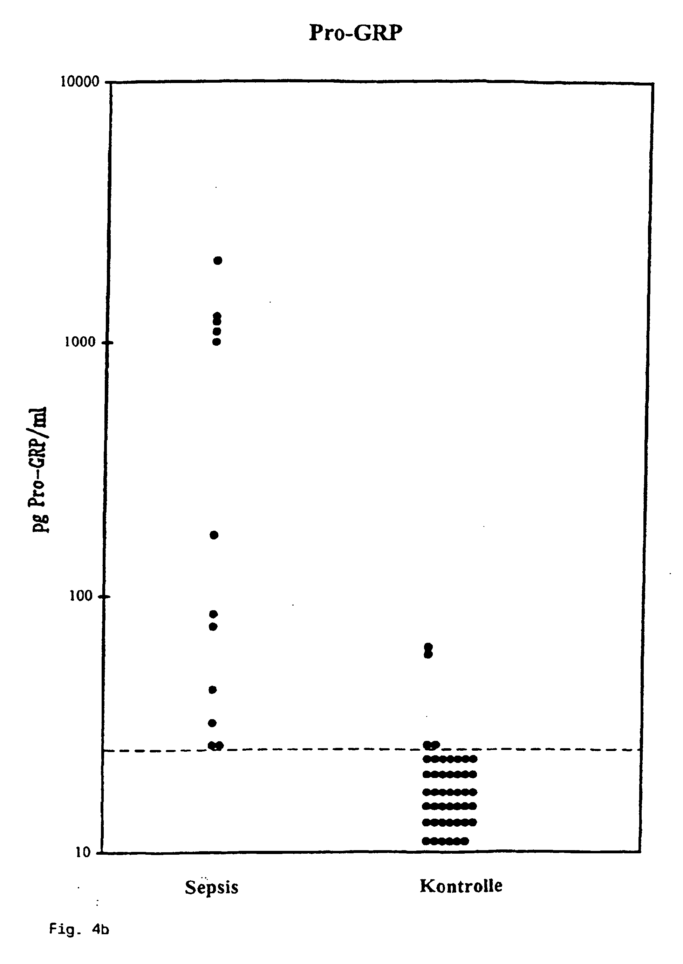

Unter Verwendung dieses Kits wurden unter Einhaltung der Arbeitsweise, die in der Gebrauchsinformation des kommerziellen Kits vorgeschrieben ist, die in Figur 4 dargestellten Meßergebnisse erhalten.

Ein Vergleich der für Procalcitonin einerseits und ProGRP andererseits erhaltenen Werte zeigt, daß bei Procalcitonin die Unterscheidung zwischen Normalseren und Sepsis-Seren zwar eindeutiger ist, daß aber die ProGRP-Konzentrationen in weitgehend ähnlicher Weise erhöht sind wie die von Procalcitonin.

### D.2. Bestimmung von pro-ANF, pro-ADM, pro-END und pro-BNP in Seren von Sepsis-Patienten und von Normalpersonen

Zur Bestimmung der (Prä-)Prohormone pro-ANF, pro-ADM, pro-END bzw. pro-BNP sind Assays der Firma DRG (DRG Instruments GmbH, D-35018 Marburg, Deutschland) in Kitform im Handel erhältlich, die für die nachfolgenden Messungen unter Beachtung der Anweisungen der Hersteller verwendet wurden.

Im einzelnen wurden verwendet:

Zur Bestimmung von pro-ANF der Prepro-ANF 26-55 (human) RIA Kit, zur Bestimmung von pro-ADM der Pro-Adrenomedullin 45-92 (human) RIA, zur Bestimmung von pro-END der Prepro-Endothelin 18-50 (human) RIA Kit, und zur Bestimmung von Pro-BNP der Prepro-BNP 22-46 (human) RIA Kit der o.g. Firma DRG.

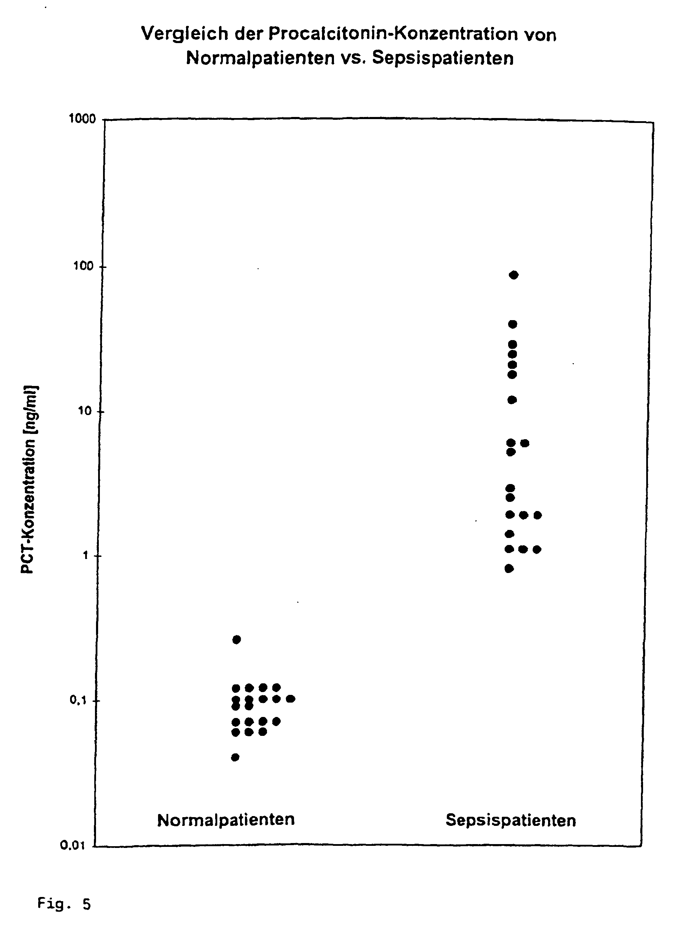

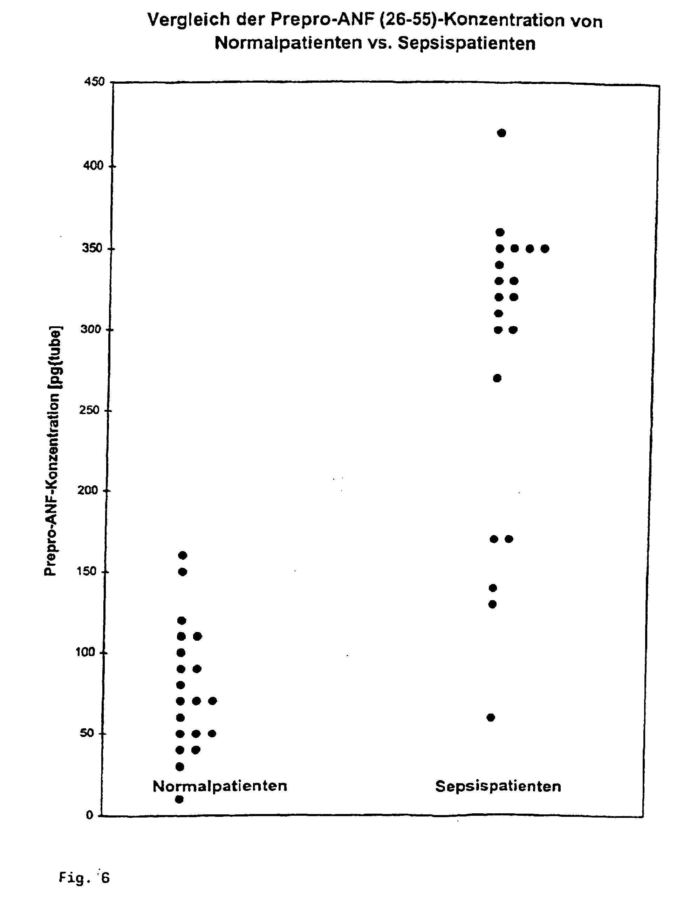

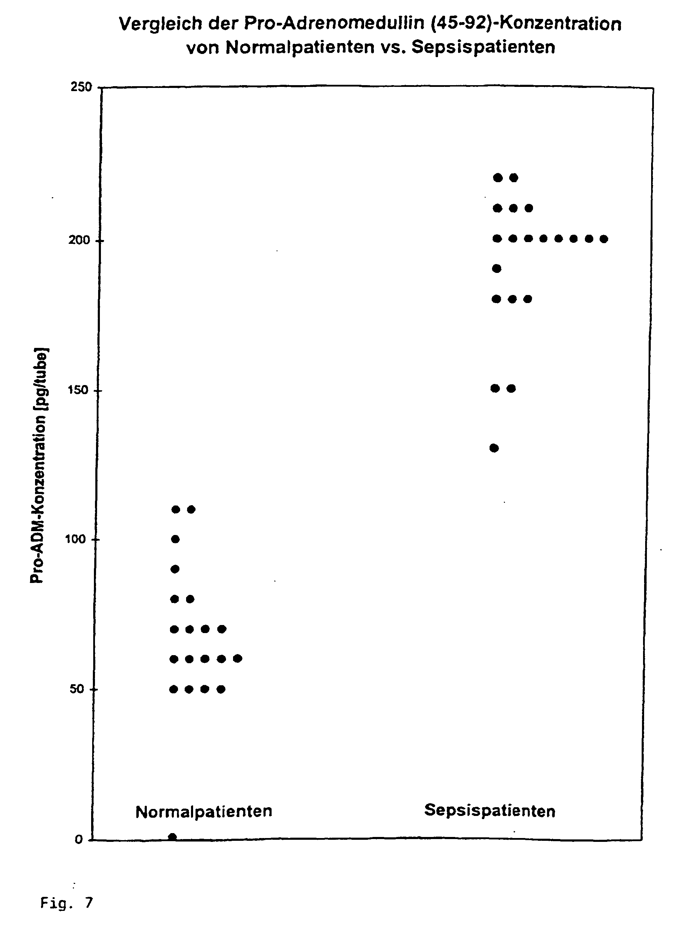

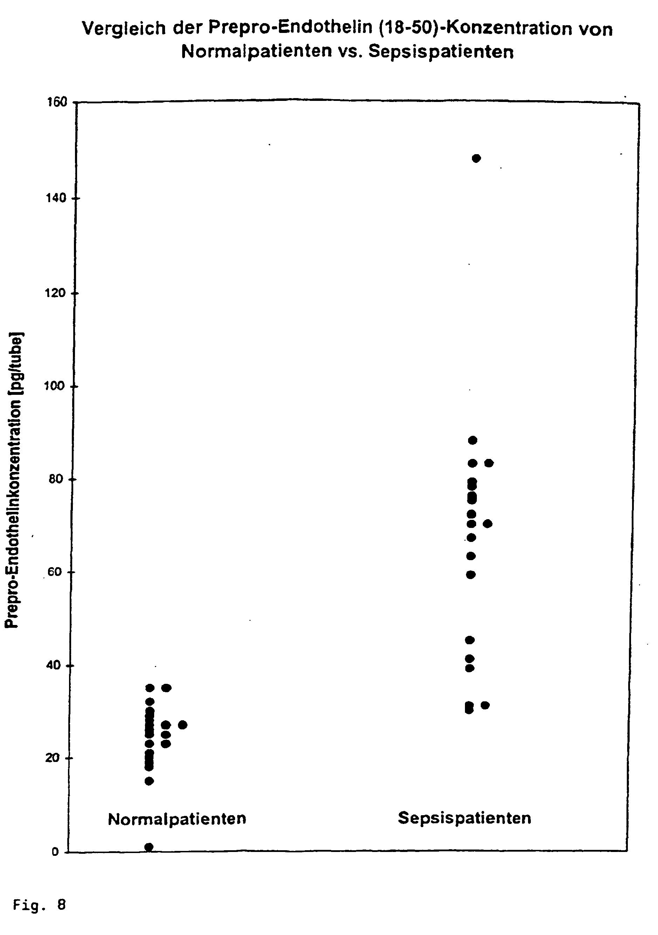

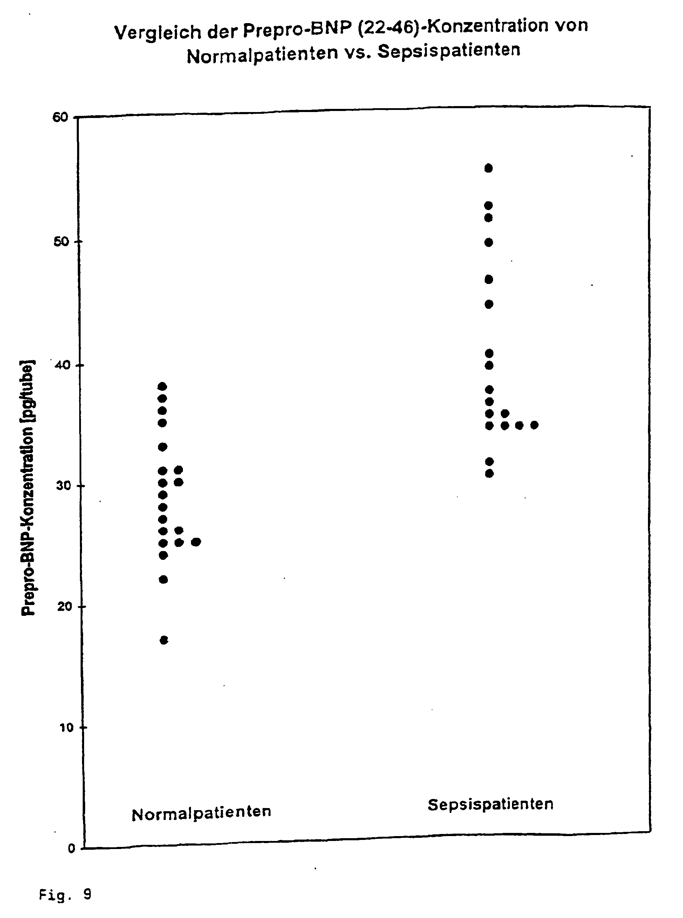

In den Seren eines Kollektivs von 20 Normalpersonen A-T sowie von 20 Sepsispatienten wurden die o.g. Prohormone sowie, parallel dazu, Procalcitonin bestimmt. Die Ergebnisse sind in der nachfolgenden Tabelle 3 zusammengefaßt. Die Daten der Tabelle 3 sind außerdem auch noch in den Figuren 5 bis 9 graphisch veranschaulicht.

Die gezeigten Ergebnisse lassen bei allen gemessenen Prohormonen bei Sepsispatienten gegenüber Normalpersonen einen mehr oder weniger klar ausgeprägten Anstieg der Werte erkennen, wenn auch die Unterscheidung von Normalpersonen und Sepsispatienten bei der Bestimmung von Procalcitonin am ausgeprägtesten ist.

Guo et al., American Journal of Respiratory Cell and Molecular Biology, Bd.1, Nr.3, Sept. 1998, S.470-476 stellt eine Beziehung zwischen pro-Endothelin-1 und einer "Upregulation" seiner Vorläufer und Sepsis her, die jedoch ausschließlich im Gewebe bzw. auf der RNA-Ebene festgestellt wurde. Im Serum ist nur eine Erhöhung von Endothelin-1, d.h. des eigentlichen Peptid-Hormons selbst, beschrieben.

Bei einer ergänzenden Literaturrecherche wurde ferner eine Veröffentlichung in J. Endocr. (1988) 119, S.159-165 ermittelt; die sich mit der Charakterisierung von-Pro-Opiomelanocortin-(POMC)-verwandten Peptiden bei septischem Schock befaßte. Es geht in der Veröffentlichung um die Frage einer erhöhten endogenen Opioid-Aktivität bei septischem Schock und deren Beeinflussung durch Verabreichung von Steroiden. Ein direkter Einfluß eines infektiösen Geschehens oder eine Beeinflussung der Meßwerte durch Antibiotika wird nicht diskutiert. Aufgrund der Fragestellung in der genannten Veröffentlichung besteht keine logische Möglichkeit zu einer verallgemeinernde Diskussion der berichteten Ergebnisse unter Einbeziehung anderer Prohormon-Peptide. Erst angesichts der hierin offenbarten Lehre der vorliegenden Erfindung läßt sich die genannte Veröffentlichung retrospektiv als weiterer Hinweis auf eine generelle Erhöhung von Prohormonen bei Sepsis interpretieren.

Die Prüfung weiterer Prohormone ist angesichts der hierin offenbarten Ergebnisse als Routinemaßnahme anzusehen, und wenn derartige Prüfungen zu positiven Ergebnissen führen und die Bestimmung derartiger weiterer Prohormone zur Sepsisdiagnose verwendet wird, wird daher von der Lehre der vorliegenden Anmeldung Gebrauch gemacht.

**Tabelle 3:**

| | PCT [ng/ml] | Pro-ANF [pg/tube] | Pro-ADM [pg/tube] | Pro-END [pg/tube] | Pro-BNP [pg/tube] |
|---|---|---|---|---|---|
| **Normalpatienten:** | | | | | |
| A | 0,07 | 69,4 | 64 | 29,3 | 31,1 |
| B | 0,26 | 26,6 | 50,2 | 15,4 | 30,1 |
| C | 0,10 | 14,7 | 1,0 | 1,0 | 24,7 |
| D | 0,06 | 53,2 | 77,8 | 19,3 | 27,4 |
| E | 0,06 | 51,1 | 66,5 | 20,6 | 25,4 |
| F | 0,04 | 95,5 | 53,5 | 28,2 | 28,9 |
| G | 0,07 | 117 | 83,5 | 18,3 | 17,3 |
| H | 0,10 | 88,1 | 52,3 | 25,1 | 28,1 |
| I | 0,10 | 69,4 | 107 | 23,2 | 25 |
| J | 0,07 | 38,3 | 91,1 | 26,1 | 25,7 |
| K | 0,06 | 111 | 64,9 | 22,8 | 29,6 |
| L | 0,09 | 73,8 | 66,3 | 32,4 | 21,6 |
| M | 0,07 | 42 | 58,9 | 27,1 | 23,7 |
| N | 0,09 | 107 | 114 | 27,3 | 31,2 |
| O | 0,10 | 56,7 | 62,5 | 20,3 | 26,2 |
| P | 0,10 | 47,2 | 51,7 | 24,5 | 33,3 |
| Q | 0,12 | 155 | 72,5 | 34,6 | 36,7 |
| R | 0,13 | 92,1 | 64,7 | 29,6 | 35,2 |
| S | 0,12 | 153 | 100 | 34,7 | 36 |
| T | 0,11 | 78 | 69,6 | 27,1 | 37,7 |

| **Sepsispatienten:** | | | | | |
|---|---|---|---|---|---|
| 13a | 1,1 | 333 | 195 | 70 | 34,9 |
| 10a | 11,9 | 62,5 | 154 | 30,5 | 30,4 |
| 18b | 6,4 | 323 | 209 | 75,4 | 46,4 |
| 19b | 21,2 | 346 | 180 | 71,6 | 50,7 |
| 8a | 1,8 | 303 | 203 | 70,3 | 48,8 |
| 9a | 24,7 | 271 | 205 | 78,4 | 33,6 |
| 9b | 29,0 | 305 | 210 | 62,5 | 40,3 |
| 12b | 5,8 | 324 | 204 | 67,4 | 36,1 |
| 10b | 1,9 | 127 | 128 | 30,5 | 33,9 |
| 16a | 86,7 | 347 | 198 | 83,3 | 39 |
| 8b | 1,1 | 138 | 153 | 29,6 | 33,7 |
| 20a | 1,4 | 167 | 176 | 41,3 | 30,9 |
| 20b | 1,1 | 170 | 178 | 39 | 34,9 |
| 13b | 0,8 | 295 | 186 | 45,3 | 36,9 |
| 19a | 17,6 | 354 | 201 | 58,6 | 54,5 |
| 7b | 2,5 | 356 | 199 | 78,6 | |
| 7a | 2,9 | 345 | 197 | 148 | |
| 16b | 40,0 | 343 | 197 | 88,1 | 43,9 |
| 12a | 5,2 | 327 | 216 | 76,1 | 34,3 |
| 15b | 1,9 | 420 | 215 | 82,5 | 52 |

## Patentansprüche

1. Verfahren zur differentialdiagnostischen Früherkennung und Erkennung, zur Beurteilung des Schweregrads und zur therapiebegleitenden Verlaufsbeurteilung von Sepsis, **dadurch gekennzeichnet, daß** man in einer Probe eines Serums aus dem Blut eines Patienten den Gehalt mindestens eines Peptid-Prohormons, das nicht Procalcitonin ist, bestimmt und aus der festgestellten Menge des bestimmten Prohormons auf das Vorliegen einer Sepsis, ihren Schweregrad und/oder den Erfolg einer therapeutischen Behandlung schließt.

2. Verfahren nach Anspruch 1, **dadurch gekennzeichnet, daß** das Peptid-Prohormon ausgewählt ist aus pro-Gastrin-Releasing Peptid (proGRP), pro-Endothelin-1 (pro-END), pro-Brain-Natriuretic-Peptid (pro-BNP), pro-Atrial-Natriuretic-Peptid (Pro-ANP bzw. pro-ANF), pro-Leptin, pro-Neuropeptid-Y, pro-Somatostatin, pro-Neuropeptid-YY oder pro-Adrenomedullin (pro-ADM).

3. Verfahren nach einem der Ansprüche 1 bis 2, **dadurch gekennzeichnet, daß** man die Bestimmung als Immunoassay oder Präzipitationsassay durchführt und auf Sepsis oder sepsis-ähnliche schwere Infektionen schließt, wenn die Konzentration des bestimmten Peptid-Prohormons signifikant über den werten liegt, die bei gesunden Normalpersonen beobachtet werden können.

4. Verfahren nach Anspruch 2, **dadurch gekennzeichnet, dass** das Prohormon pro-ANF mit einem Prepro-ANF 26-55 (human) RIA Kit bestimmbar ist.

5. Verfahren nach Anspruch 2, **dadurch gekennzeichnet, dass** das Prohormon pro-ADM mit einem Pro-Adrenomedullin 45-92 (human) RIA Kit bestimmbar ist.

6. Verfahren nach Anspruch 2, **dadurch gekennzeichnet, dass** das Prohormon pro-END mit einem Prepro-Endothelin 18-50 (human) RIA Kit bestimmbar ist.

7. Verfahren nach Anspruch 2, **dadurch gekennzeichnet, dass** das Prohormon pro-BNP mit einem Prepro-BNP 22-46 (human) RIA Kit bestimmbar ist.

## Claims

1. A method for the differential-diagnostic early detection and detection, for the assessment of the severity, and for the monitoring of the treatments accompanied by therapy of a sepsis, **characterized in that** the level of at least one peptide prohormone other than procalcitonin is determined in a blood serum sample from a patient, and conclusions are drawn with respect to the presence of a sepsis, its severity and/or the success of a therapeutic treatment from the detected amount of the determined prohormone.

2. The method according to Claim 1, **characterized in that** the peptide prohormone is selected from pro-gastrin-releasing peptide (proGRP), pro-endothelin-1 (pro-END), pro-brain-natriuretic peptide (pro-BNP), pro-atrial-natriuretic peptide (pro-ANP or pro-ANF), pro-leptin, pro-neuropeptide-Y, pro-somatostatin, pro-neuropeptide-YY or pro-adrenomedullin (pro-ADM).

3. The method according to any of Claims 1 or 2, **characterized in that** said determination is carried out as an immunoassay or precipitation assay, and a diagnosis of the presence of sepsis or severe sepsis-like infections is made if the concentration of the peptide prohormone determined is significantly higher than the levels observed in healthy normal individuals.

4. The method according to claim 2, **characterized in that** the prohormone proANF can be determined with a pre-proANF 26-55 (human) RIA kit.

5. The method according to claim 2, **characterized in that** the prohormone proADM can be determined with a pro-adrenomedullin 45-92 (human) RIA kit.

6. The method according to claim 2, **characterized in that** the prohormone proEND can be determined with a pre-pro-endothelin 18-50 (human) RIA kit.

7. The method according to claim 2, **characterized in that** the prohormone proBNP can be determined with a pre-proBNP 22-46 (human) RIA kit.

## Revendications

1. Un procédé de dépistage précoce et de dépistage, par diagnostic différentiel, pour évaluer le degré de gravité et l'évolution thérapeutique d'une septicémie, **caractérisé en ce que** l'on détermine dans un échantillon d'un sérum du sang d'un patient la teneur en au moins une prohormone peptidique qui n'est pas la procalcitonine et que l'on infère l'existence d'une septicémie, son degré de gravité et/ou le résultat d'un traitement thérapeutique, à partir de la quantité de la prohormone déterminée.

2. Le procédé selon la revendication 1, **caractérisé en ce que** la prohomone peptidique est choisie parmi le pro-peptide libérant la gastrine (proGRP: pro-Gastrin-Releasing-Peptid), la pro-endothéline-1 (pro-END), le pro-peptide natriurétique cérébral (pro-BNP: pro-Brain-Natriuretic-Peptid), le pro-peptide natriurétique ventriculaire (pro-ANP, respectivement pro-ANF), la pro-leptine, le pro-neuropeptide-Y, la pro-somatostatine, le pro-neuropeptide-YY ou la pro-adrénomédulline (pro-ADM).

3. Le procédé selon l'une des revendications 1 à 2, **caractérisé en ce que** la détermination est effectuée en tant que test immunologique ou test de précipitation et que l'on infère une septicémie ou une infection grave analogue à une septicémie, lorsque la concentration de la prohormone peptidique déterminée se situe de manière significative au-dessus des valeurs qui peuvent être attendues chez des personnes normales en bonne santé.

4. Procédé selon la revendication 2, **caractérisé en ce que** la prohormone pro-ANF peut être déterminée avec un kilt de dosage radio-immunologique (RIA) de pré-pro-ANF 26-55 (humain).

5. Le procédé selon la revendication 2, **caractérisé en ce que** la prohormone pro-ADM peut être déterminée avec un kit de dosage radio-immunologique de pro-adrénomédulline 45-92 (humaine).

6. Le procédé selon la revendication 2, **caractérisé en ce que** la prohormone pro-END peut être déterminée avec un kit de dosage radio-immunologique de pré-pro-endothéline 18-50 (humaine).

7. Le procédé selon la revendication 2, **caractérisé en ce que** la prohormone pro-BNP peut être déterminée avec un kit de dosage radio-immunologique de pré-pro-BNP 22-46 (humain).
